# EUROPÄISCHE PATENTSCHRIFT

(11) **EP 4 054 664 B1**
(45) Veröffentlichungstag und Bekanntmachung des Hinweises auf die Patenterteilung: **18.10.2023**
(21) Anmeldenummer: 20811527.9
(22) Anmeldetag: 02.11.2020
(51) Int. Cl.: A61L 29/08, A61L 29/14, A61L 29/16, A61M 25/10

(54) **BALLONKATHETER MIT HYDROGEL-BESCHICHTUNG**
BALLOON CATHETER WITH HYDROGEL COATING
CATHÉTER À BALLONNET POURVU D'UN REVÊTEMENT D'HYDROGEL

(30) Priorität: 04.11.2019 DE 102019129596
(43) Veröffentlichungstag der Anmeldung: 14.09.2022
(73) Patentinhaber: Ruebben, Alexander, 98000 Monaco (MC)
(72) Erfinder: Ruebben, Alexander, 98000 Monaco (MC)
(74) Vertreter: Schneiders & Behrendt Bochum
(86) Internationale Anmeldenummer: PCT/EP2020/080655
(87) Internationale Veröffentlichungsnummer: WO 2021/089470

(56) Entgegenhaltungen:
- EP-A1- 3 228 348
- WO-A1-2016/050303

## Beschreibung

Die Erfindung betrifft einen Ballonkatheter zur Einführung in das Blutgefäßsystem des menschlichen oder tierischen Körpers mit einem sich in Längsrichtung erstreckenden Schaft, wobei der Ballonkatheter über einen distalen Abschnitt verfügt, in dem ein Ballon angeordnet ist, der durch Zufuhr eines Fluids über ein durch den Schaft verlaufendes Lumen expandierbar ist.

Der Einsatz von Ballonkathetern gehört heutzutage zum Standard im klinischen Alltag. Ihre Verwendung im Rahmen von intravaskulären Interventionen bezieht sich meist auf die Aufweitung verengter Gefäßstellen, entweder mit Hilfe des Ballonkatheters an sich oder in Kombination mit weiteren Medizinprodukten wie beispielsweise ballonexpandierbaren Stents. Bei der perkutanen transluminalen Angioplastie wird ein Ballonkatheter über einen Führungsdraht und einen Führungskatheter an den Ort der Stenose gebracht und durch Zufuhr eines Fluids unter Druck (ca. 4 bis 12 bar) expandiert. Im Bereich der Stenose vorhandene Ablagerungen werden in die Gefäßwandung gedrückt. Zusätzlich kann zwecks dauerhafter Offenhaltung des Blutgefäßes ein Stent (Gefäßendoprothese) gesetzt werden. Um das erneute Auftreten einer Stenose durch ein gefäßverengendes Überwuchern der erweiterten Stelle zu verhindern, können auch medikamentenbeschichtete Ballonkatheter eingesetzt werden, die bei der Expansion einen Wirkstoff wie Paclitaxel an der Stelle der Gefäßverengung abgeben. Nach erfolgter Behandlung und dem anschließenden Zusammenfalten des Ballons wird der Ballonkatheter aus dem Gefäßsystem zurückgezogen und entfernt.

Als Zugangsstelle für Ballonkatheter, aber auch andere Katheter, beispielsweise zum Setzen eines Stents oder zur Einbringung anderer Implantate in das Blutgefäßsystem, wird häufig die Arteria femoralis im Bereich der Leiste gewählt. Die Arteria femoralis verläuft zumindest partiell relativ oberflächlich und ist daher für den behandelnden Arzt gut zugänglich. Darüber hinaus lassen sich über die Arteria femoralis (Leistenarterie) verhältnismäßig großlumige Katheter mit großen Außendurchmessern einbringen.

Da der Katheter von der Zugangsstelle bis zur Zielposition, die beispielsweise im Bereich des Herzens oder des Gehirns liegen kann, über verhältnismäßig weite Strecken vorgeschoben werden muss, ist ein geringer Reibungswiderstand von Vorteil. Bei transürethralen Foley-Kathetern ist es bekannt, diese unmittelbar vor dem Einsatz mit einem Hydrogel zu beschichten, das die Gleitfähigkeit des Katheters beim Einführen in die Harnröhre erhöht. Gleichzeitig wird in der Regel auch die Harnröhre selbst mit dem Hydrogel versehen. Hier handelt es sich jedoch nicht um Katheter, die in das Blutgefäßsystem eingeführt werden, bei derartigen Kathetern ist eine Vorabbeschichtung mit einem Hydrogel nicht üblich.

Zum Teil sind aus dem Stand der Technik, beispielsweise der WO 2017/139357 A1, auch Ballonkatheter bekannt, bei denen der Ballon selbst eine Hydrogelbeschichtung aufweist, in die ein über den Ballon applizierbarer Wirkstoff eingebettet ist. In diesem Fall dient das Hydrogel jedoch nicht als Mittel zur Erhöhung der Gleitfähigkeit des Ballonkatheters, sondern als Matrix für den abzugebenden Wirkstoff.

Ausgehend vom vorgeschriebenen Stand der Technik stellt sich somit die Aufgabe, einen Ballonkatheter zur Verfügung zu stellen, der sich besonders gut auch über verhältnismäßig große Distanzen und durch englumige Blutgefäße an die Zielposition vorschieben lässt.

Diese Aufgabe wird erfindungsgemäß gelöst durch einen Ballonkatheter zur Einführung in das Blutgefäßsystem des menschlichen oder tierischen Körpers mit einem sich in Längsrichtung erstreckenden Schaft, wobei der Ballonkatheter über einen distalen Abschnitt verfügt, in dem ein Ballon angeordnet ist, der durch Zufuhr eines Fluids über ein durch den Schaft verlaufendes Lumen expandierbar ist, wobei die distale Spitze des Ballonkatheters eine Beschichtung mit einem Hydrogel aufweist und sich in Längsrichtung betrachtet der Bereich der Beschichtung mit dem Hydrogel vom distalen Ende des Ballonkatheters bis maximal zur Mitte des Ballons erstreckt.

Anders als bei den zuvor beschriebenen Ballonkathetern wird erfindungsgemäß somit das Hydrogel auf die distale Spitze des Ballonkatheters aufgebracht und nicht oder jedenfalls nicht vorrangig auf den Ballon selbst. Durch die Aufbringung auf die distale Spitze wird erreicht, dass sich der Ballonkatheter gut vorschieben lässt, auch dann, wenn im Blutgefäßsystem Engstellen auftreten. Beim Passieren einer solchen Engstelle wird vielmehr das auf die distale Spitze des Ballonkatheters aufgebrachte Hydrogel weiter in Richtung proximal gedrängt, so dass der Ballonkatheter distal über eine besonders gute Gleitfähigkeit verfügt.

Das Hydrogel kann in einer Form aufgebracht werden, dass es zunächst an der distalen Spitze des Ballonkatheters lokalisiert bleibt. Wenn das Hydrogel beim Einführen des Ballonkatheters in das Blutgefäßsystem mit Blut in Kontakt kommt, quillt es durch Wasseraufnahme auf und bildet das sehr gut gleitfähige Gel, wobei unter Umständen eine partielle Peptisation und damit ein Gel-Sol-Übergang stattfindet.

Durch die Aufbringung des Hydrogels wird nicht nur das Vorschieben des Ballonkatheters im Blutgefäß erleichtert, sondern auch das Einführen des Ballonkatheters in eine Einführhilfe oder Einführschleuse, die zumeist im Bereich der Arteria femoralis platziert wird. Derartige Katheter-Einführhilfen sind zumeist aus einem Kunststoffmaterial gefertigt und verfügen häufig über eine Membran, die vom vorzuschiebenden Ballonkatheter zunächst durchstoßen werden muss.

Der Bereich der Beschichtung mit dem Hydrogel erstreckt sich vom distalen Ende des Ballonkatheters bis maximal zur Mitte des Ballons. Vorzugsweise ist die Beschichtung mit dem Hydrogel auf die distale Spitze des Ballonkatheters beschränkt. Insbesondere soll der Ballonkatheter nicht oder nur partiell mit dem Hydrogel versehen sein. Es kann zwar über die unmittelbare distale Spitze hinaus auch ein Teil des Ballonkatheters mit dem Hydrogel beschichtet sein, diese Beschichtung erstreckt sich jedoch vom distalen Ende aus gesehen bis maximal zur Mitte des Ballons, vorzugsweise nur über das distale Drittel des Ballons. Weiter vorzugsweise erstreckt sich die Beschichtung mit dem Hydrogel lediglich über das am weitesten distal liegende Viertel oder das am weitesten distal liegende Fünftel des Ballons, wobei jeweils die Längsrichtung des Ballonkatheters betrachtet wird.

Die Länge des mit dem Hydrogel beschichteten Bereichs des Ballonkatheters kann beispielsweise max. 10 cm, bevorzugt max. 5 cm, weiter bevorzugt max. 3 cm, weiter bevorzugt max. 2 cm, weiter bevorzugt max. 1 cm betragen, wobei jeweils die Strecke vom distalen Ende des Ballonkatheters in Richtung proximal betrachtet wird.

Dadurch, dass lediglich die distale Spitze des Ballonkatheters mit dem Hydrogel beschichtet wird, wird die Gesamtmenge an aufgebrachtem Hydrogel gering gehalten. Auf der anderen Seite erfüllt die distal aufgebrachte Hydrogelbeschichtung ihre Funktion, denn für die Verbesserung der Vorschiebbarkeit innerhalb eines Blutgefäßes ist in erster Linie die distale Spitze des Ballonkatheters von Bedeutung. Sobald die distale Spitze mit dem sich unmittelbar anschließenden Ballonabschnitt eine Engstelle überwunden hat, folgen die weiteren Bereiche des Ballonkatheters normalerweise unproblematisch. Darüber hinaus ist zu berücksichtigen, dass an der distalen Spitze aufgebrachtes Hydrogel nach Aktivierung durch Aufnahme von Wasser eine Konsistenz aufweist, dass es gerade durch das Passieren der Engstelle in gewissem Maße in Richtung proximal verteilt wird, sodass der Ballonkatheter im distalen Abschnitt insgesamt eine gute Gleitfähigkeit aufweist.

Die Menge an aufgebrachtem Hydrogel sollte so gewählt werden, dass sie einerseits ihre Funktion der Verbesserung der Vorschiebbarkeit des Ballonkatheters erfüllen kann, auf der anderen Seite jedoch so gering wie möglich ist. Das Hydrogel stellt einen Zusatzstoff dar, der nur in möglichst geringer Menge in das Blutgefäßsystem eingebracht werden sollte. Von einigen als Hydrogel verwendbaren Stoffen ist beispielsweise bekannt, dass sie in größeren Mengen Nebenwirkungen oder allergische Reaktionen bis hin zu einem anaphylaktischen Schock herbeiführen können Gerade weil die Menge an aufgebrachter Beschichtung relativ klein gehalten werden sollte, ist es vorteilhaft, lediglich die distale Spitze des Ballonkatheters bzw. lediglich kleine Bereiche des Ballonkatheters nahe dem distalen Ende zu beschichten. Vorzugsweise liegt die Gesamtmenge an aufgebrachtem Hydrogel bei ≤ 5 mg, weiter vorzugsweise ≤ 3 mg, weiter vorzugsweise ≤ 1 mg. Die Hydrogelbeschichtung ist in der Regel frei von Wirkstoff.

Die Hydrogelbeschichtung pro Fläche beträgt zweckmäßigerweise ≤ 2,5 µg/mm², weiter vorzugsweise 0,5 bis 2,5 µg/mm², wobei jeweils nur die mit Hydrogel beschichtete Fläche des Ballonkatheters betrachtet wird. Auf diese Weise wird ein ausreichender Effekt erzielt, ohne dabei Gefahr zu laufen, dass eine zu große Menge des Hydrogels abgetragen und vom Blut aufgenommen wird.

Für das Hydrogel können übliche für Hydrogele verwendbare Stoffe eingesetzt werden, insbesondere Polysaccharide, modifizierte Polysaccharide, Methacrylate, Polypeptide, Proteine oder auch synthetische Polymere. Als modifizierte Polysaccharide werden insbesondere solche Polysaccharide verstanden, die bzw. deren Monomereinheiten in besonderer Weise funktionalisiert sind. Beispielsweise können einzelne Monomerbausteine über Amino- oder Amidfunktionen, Carboxyfunktionen o. ä. verfügen oder auch partiell oxidiert sein. Beispiele sind etwa Polyaminosaccharide wie Chitosan oder Hyaluronsäure mit einer N-Acetylaminofunktion und Glucoronsäureeinheiten. Die Hydrogele können quervernetzt oder nicht quer vernetzt sein.

Insbesondere kann das Hydrogel enthalten: Dextran, Polyhydroxyethylmethacrylat, ein Polyacrylat, Chitosan, oxidierte Cellulose, Polyvinylpyrrolidon, Polyethylenglycol, Hyaluronsäure, Agarose, Stärke, Stärkederivate wie Hydroxyethylstärke, ein Poloxamer, Gelatine, Fibrin, Polyvinylalkohol, Propylenglycol, Polyacrylsäure, Methylcellulose, Carboxymethylcellulose, Hydroxypropylmethylcellulose, Hydroxyethylcellulose, Hydroxypropylcellulose, Alginat, Amylose, Amylopektin, Xanthan, Xyloglucan, Peptidoglycan, Polyoxyethylenderivate, ein Silikon und/oder Elastin. Bevorzugt sind Polysaccharide.

Unter proximal wird im Rahmen der Erfindung in Richtung Körperäußeres, d. h. zum behandelnden Arzt hin verstanden, unter distal die entgegengesetzte Richtung, d. h. in Richtung Zielposition. Die Längsrichtung ist die Richtung von proximal in Richtung distal bzw. umgekehrt. Unter radial wird die Ebene senkrecht zur Längsachse des Ballonkatheters verstanden.

Typischerweise erstreckt sich durch den Schaft zumindest teilweise ein zweites Lumen in Längsrichtung, das der Aufnahme eines Führungsdrahts dient. Dieses Lumen kann sich bis zum distalen Ende des Ballonkatheters erstrecken, d. h. der Führungsdraht kann am distalen Ende des Ballonkatheters aus diesem austreten. Entsprechend kann zunächst ein Führungsdraht an die gewünschte Zielposition gebracht werden, bevor der Ballonkatheter selbst über den Führungsdraht an die Zielposition vorgeschoben wird.

In diesem Zusammenhang sind im Wesentlichen zwei unterschiedliche Systeme aus dem Stand der Technik bekannt, nämlich Over-The-Wire (OTW)- und Rapid Exchange (Rx)-Ballonkatheter. Der erfindungsgemäße Ballonkatheter kann sowohl als OTW- als auch als Rx-Ballonkatheter vorliegen. Während sich bei einem OTW-Katheter das Lumen für den Führungsdraht über die gesamte Länge des Katheters von proximal nach distal erstreckt, verfügt der Rx-Katheter über eine separate Zuführöffnung für den Führungsdraht (Rx-Port), an der der Führungsdraht deutlich distal des proximalen Endes des Katheters aus dem Katheter austritt. Entsprechend laufen die Lumen für die Fluidzufuhr und den Führungsdraht im Falle eines OTW-Ballonkatheters konzentrisch oder parallel zueinander vom proximalen Ende des Katheters bis zum Ballon, während dies bei einem Rx-Katheter nur zwischen Rx-Port und Ballon der Fall ist. Der Abschnitt zwischen Rx-Port und proximalem Ende hingegen weist nur ein Lumen für die Fluidzufuhr auf. Die Lumen können in den Bereichen, in denen der Katheter zwei Lumen aufweist, konzentrisch verlaufen, d. h. das engere innere Lumen für den Führungsdraht verläuft durch das weitere äußere Lumen für die Fluidzufuhr.

Unter Ballon im Sinne der Erfindung wird das durch Zufuhr eines Fluids expandierbare Element eines Ballonkatheters verstanden, unabhängig davon, welche Form das expandierbare Element aufweist oder aus welchem Material es besteht. Ballonkatheter sind grundsätzlich hinlänglich im Stand der Technik bekannt und weisen einen langgestreckten, von proximal nach distal verlaufenden Schaft sowie einen im distalen Abschnitt angeordneten Ballon auf. Es handelt sich um einen Katheter, der hinsichtlich seiner Dimensionen auf die Einführung in das jeweilige Blutgefäßsystem abgestimmt ist. Dabei können die exakten Dimensionen variieren, je nachdem, ob das Blutgefäß bspw. eine Koronararterie, ein intrakranielles Blutgefäß oder eine Unterschenkelarterie ist.

Darüber hinaus verfügt der Ballonkatheter über Mittel zur Zuführung eines Fluids zum Ballon in Form eines Zufuhrlumens, das sich über die Länge des Ballonkatheters erstreckt. Die durch den Schaft verlaufenden Lumen werden normalerweise jeweils durch einen schlauch- oder rohrförmigen Tubus gebildet.

Der Begriff Tubus wird in diesem Zusammenhang im Sinne einer Röhre oder eines Schlauchs verwendet, der sich zumindest teilweise durch den Ballonkatheter in Längsrichtung erstreckt und ein durch das Innere des Tubus verlaufendes Lumen aufweist. Dabei kann der Tubus die Form eines Hohlzylinders mit kreisförmigem oder elliptischem Querschnitt aufweisen, dies ist jedoch nicht unbedingt erforderlich. Denkbar sind im Querschnitt betrachtet auch nahezu beliebige andere Formen. Ein kreisförmiger oder auch elliptischer Querschnitt weist allerdings den Vorteil auf, dass ein Tubus gut durch den anderen Tubus hindurch geführt werden kann, wobei in der Regel der zweite Tubus durch den ersten Tubus gelegt wird.

Der Ballon kann, im Falle eines compliant oder semi-compliant Ballons, mindestens teilweise aus einem elastischen Material gefertigt sein. Als elastisches Material kann z. B. ein Polyurethan, ein Polyolefincopolymer, ein Polyethylen oder ein Silikon verwendet werden. Weitere verwendbare Materialien sind thermoplastische Elastomere, insbesondere Polyetherblockamide (PEBA). Hierbei handelt es sich um ein thermoplastisches Elastomer, das durch Polykondensation eines Carbonsäurepolyamids mit einem Polyether mit endständigen OH-Gruppen erhältlich ist. Insbesondere wird PEBA unter der Bezeichnung PEBAX^{®} von der Firma Arkema vertrieben. Auch Polyamide wie Nylon (Polyhexamethylenadipinsäureamid) oder solche Polyamide, wie sie unter der Bezeichnung Grilamid^{®} von der Firma EMS-GRIVORY vertrieben werden, lassen sich verwenden.

Der Druck, mit dem der Ballon zur Expansion im Blutgefäß beaufschlagt wird, liegt typischerweise zwischen 4 und 12 bar, bevorzugt 6 bis 8 bar. Als Fluid kann bspw. mit Kontrastmittel versetztes Wasser oder eine mit Kontrastmittel versetzte Kochsalzlösung dienen. Die Dimensionen des Ballons können je nach Anwendungsbereich stark differieren, der Durchmesser im expandierten Zustand kann beispielsweise zwischen ca. 1 und ca. 50 mm liegen, die Länge zwischen ca. 5 und ca. 300 mm. Ggf. können die Maße hiervon aber auch abweichen, beispielsweise bei Einsatz des Ballons/Ballonkatheters in der Veterinärmedizin.

Der Schaft des Ballonkatheters kann aus üblichen Materialien gefertigt sein, wobei auch unterschiedliche Materialien verwendet werden können, beispielsweise um den distalen Abschnitt weicher auszubilden als den proximalen Abschnitt. Gängige Materialien sind Polymere wie Polyethylen, Polyurethan, Polyvinylchlorid, Polyamide, Polyimide, Silikone, Polyetheramide, Polytetraflurethylen, EPDM (Ethylen-Propylen-Dien-Kautschuk), Polyetherblockamide (PEBA) oder Polyamide, wie sie unter der Bezeichnung Grilamid^{®} von der Firma EMS-GRIVORY vertrieben werden. Ggf. können insbesondere proximal liegende Bereiche des Schafts auch aus Metall, bspw. Edelstahl gefertigt sein.

Die Länge des Schafts, einschließlich des Bereichs, in dem der Ballon angeordnet ist, jedoch ohne proximal an den Schaft angeordnete Hubs oder ähnliche Anschlüsse, beträgt typischerweise mindestens 160 cm, vorzugsweise mindestens 165 cm. Typisch ist eine Länge zwischen 160 und 180 cm.

Der erfindungsgemäße Ballonkatheter kann auch der Platzierung eines Stents (Endoprothese) im Körperlumen dienen. Stents sind röhrenartige Stützstrukturen, die in einem Blutgefäß implantiert werden, um dieses dauerhaft offen zu halten. Derartige Stents können selbstexpandierend sein oder mit Hilfe eines Ballons expandiert werden. Hierzu wird der Stent auf dem Ballon aufgecrimpt und mit Hilfe des Ballonkatheters in das Blutgefäß eingebracht. An der vorgesehenen Stelle wird sodann der Ballon durch Zufuhr eines Fluids expandiert, wodurch sich auch der Stent weitet und im Blutgefäß verankert wird. Gleichzeitig wird bei Verwendung des erfindungsgemäßen Ballons der Wirkstoff an die Wandung des Blutgefäßes abgegeben. Schließlich wird der Ballon wieder zusammengezogen und aus dem Blutgefäß entfernt, während der Stent im Blutgefäß verbleibt.

Bei Bedarf kann der Ballonkatheter einen mit einem Wirkstoff beschichteten Ballon aufweisen, auch Drug Eluting Balloons genannt. Der Wirkstoff ist vorzugsweise ausgewählt aus der Gruppe: Tretinoin, Orphanrezeptoragonisten, Elafinderivate, Corticosteroide, Steroidhormone, Paclitaxel, Rapamycin (Sirolimus), Tacrolimus, hydrophobe Proteine, Heparin und/oder hormonartige oder zellproliferationsverändernde Substanzen. Besonders bevorzugt sind Paclitaxel, Tacrolimus und Sirolimus.

Am proximalen Ende des Ballonkatheters, sich anschließend an den Schaft, ist zumeist ein sog. Katheterhub vorgesehen, d. h. ein Anschlussstück für die Vorrichtung zur Fluidzufuhr und Druckbeaufschlagung. Die Verbindung kann z. B. eine herkömmliche Luer- bzw. Luer-Lock-Verbindung sein. Insbesondere ist es sinnvoll zwei Luer-Lock-Anschlüsse, typischerweise weibliche Anschlüsse vorzusehen, von denen einer dem Anschluss des ersten Lumens an einen Ballondilatator und ein weiterer der Einführung des Führungsdrahts in den Ballonkatheter dient. Die Anschlüsse können beispielsweise aus einem Polycarbonat gefertigt sein. Der durch den Ballonkatheter verlaufende Führungsdraht kann an seinem proximalen Ende durch einen Torquer gehalten werden, was die Handhabung des in der Regel sehr dünnen Führungsdrahts erleichtert.

Typischerweise liegt der deflatierte Ballon des Ballonkatheters in einem gefalteten Zustand vor. Je nach Größe des Ballons können dabei unterschiedlich viele Falten ausgebildet sein, die anschließend um die Achse des Katheters in gleicher Richtung aufgewunden werden. Hierdurch wird eine deutliche Reduktion des Durchmessers erreicht. Um den Durchmesser weiter zu reduzieren, werden im Stand der Technik verschiedene Methoden vorgeschlagen. Beispielhaft sei hier das sogenannte Cut-Back-Verfahren erwähnt, bei dem der gefaltete Ballon mit übergezogenem Schlauch durch eine in der Regel trichterartige Düse geführt wird. Der Schlauch wird hierdurch gestreckt und verringert seinen Durchmesser, wodurch sich folglich auch der Durchmesser des in dem Schlauch befindlichen Ballons verringert. Ein solches Verfahren wird in der WO 2016/050303 A1 beschrieben. In diesem Dokument wird ein Ballonkatheter beschrieben, der im distalen Abschnitt über eine größere Flexibilität verfügt als im proximalen Abschnitt. Auf diese Weise kann der Ballonkatheter auch englumigen Gefäßen gut folgen, gleichwohl ist er aber über längere Distanzen vorschiebbar. Diese Eigenschaften werden durch die Erfindung weiter verbessert.

Entlang des Ballonkatheters können an verschiedenen Positionen röntgendichte Markierungen angebracht sein, die der Visualisierung des Katheters im Röntgenbild dienen. Insbesondere kann es sich dabei um Markierungen aus Platin oder einer Platinlegierung handeln.

Die Erfindung wird anhand der beigefügten Figuren beispielhaft näher erläutert. Es zeigen:
- Fig. 1:: Einen erfindungsgemäßen Ballonkatheter in der Seitenansicht;
- Fig. 2: den distalen Teil des Schafts des Ballonkatheters aus Fig. 1 im Längsschnitt.

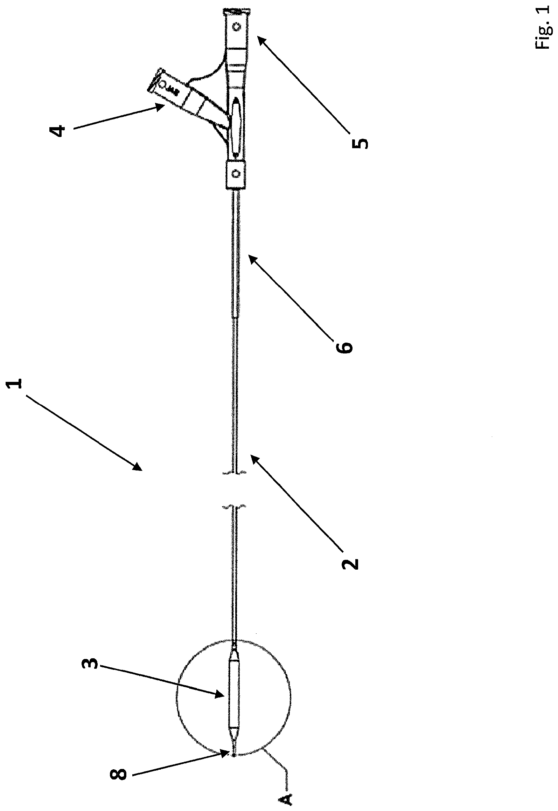

In Fig. 1 ist der erfindungsgemäße Ballonkatheter 1 in der Seitenansicht dargestellt, wobei in der hier gewählten Darstellung rechts proximal und links distal bedeutet. Der Ballonkatheter 1 weist einen sich in Längsrichtung erstreckenden Schaft 2 auf, dessen Außendurchmesser im hier nur verkürzt dargestellten proximalen Abschnitt 6 größer ist als weiter distal. Der Abschnitt des Schafts 2, in dem der Ballon 3 angeordnet ist, ist mit dem Buchstaben A gekennzeichnet. Der Ballon 3 ist im nicht expandierten, jedoch nicht zusammengefalteten Zustand dargestellt. Durch den Schaft 2 verlaufen (hier nicht dargestellt) ein erstes Lumen für die Fluidzufuhr sowie ein zweites Lumen zur Aufnahme des Führungsdrahts, wobei die beiden Lumen jeweils durch einen Tubus gebildet werden. Distal des Ballons 3 erkennt man die distale Spitze 8.

Proximal schließen sich an den proximalen Abschnitt 6 des Schafts 2 zwei Katheterhubs in Form von Luer-Lock-Verbindungen 4, 5 an, wobei die Verbindung 4 der Fluidzufuhr in das erste Lumen mittels eines Ballondilatators und die Verbindung 5 der Einführung des Führungsdrahts in das zweite Lumen dienen.

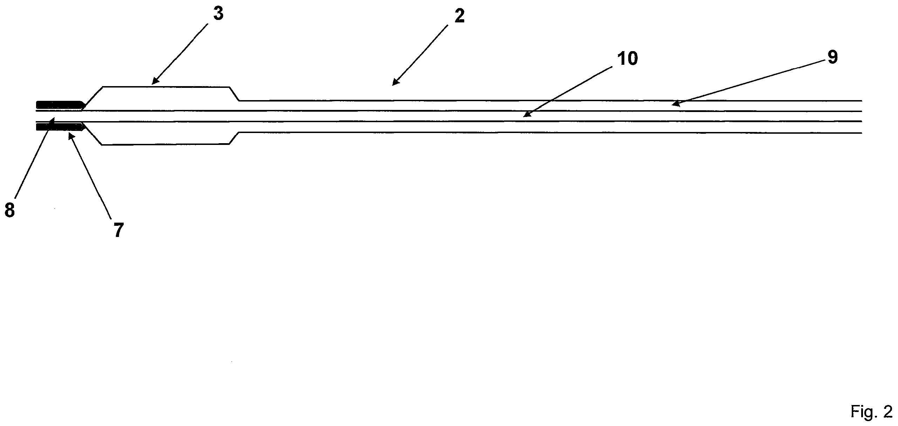

In Fig. 2 ist lediglich der distale Teil des Schafts 2 dargestellt. Ein erstes Lumen 9 ist so gestaltet, dass es das zweite Lumen 10 aufnehmen kann, d. h. das zweite Lumen 10 verläuft in Längsrichtung durch das erste Lumen 9. Das erste Lumen 9 steht an seinem distalen Ende mit dem Ballon 3 in Verbindung, welcher durch Fluidzufuhr durch das erste Lumen 9 expandiert werden kann. Das zweite Lumen 10 hingegen verfügt am distalen Ende über eine Öffnung und dient der Aufnahme des hier nicht dargestellten Führungsdrahts.

Am distalen Ende des Schafts 2 befindet sich die distale Spitze 8. Diese ist radial, d. h. über den Umfang mit einem Hydrogel 8 beschichtet. Die Beschichtung mit dem Hydrogel 8 kann sich geringfügig bis über den Ballon 3 erstrecken, dies ist jedoch nicht notwendig; vielmehr ist es von Vorteil, wenn die Hydrogelbeschichtung 8 im Wesentlichen auf die distale Spitze 8 beschränkt bleibt. Wenn der Ballonkatheter 1 eine Engstelle im Blutgefäß passiert, verteilt sich die Hydrogelbeschichtung 8 automatisch partiell weiter in Längsrichtung nach proximal und eine gute Gleitfähigkeit und Vorschiebbarkeit des Ballonkatheters 1 ist gewährleistet.

## Patentansprüche

1. Ballonkatheter zur Einführung in das Blutgefäßsystem des menschlichen oder tierischen Körpers mit einem sich in Längsrichtung erstreckenden Schaft (2), wobei der Ballonkatheter (1) über einen distalen Abschnitt (A) verfügt, in dem ein Ballon (3) angeordnet ist, der durch Zufuhr eines Fluids über ein durch den Schaft (2) verlaufendes Lumen (9) expandierbar ist, wobei die distale Spitze (8) des Ballonkatheters (1) eine Beschichtung mit einem Hydrogel (7) aufweist,
**dadurch gekennzeichnet,**
**dass** sich in Längsrichtung betrachtet der Bereich der Beschichtung mit dem Hydrogel (7) vom distalen Ende des Ballonkatheters (1) bis maximal zur Mitte des Ballons (3) erstreckt.

2. Ballonkatheter nach Anspruch 1, **dadurch gekennzeichnet, dass** der Bereich der Beschichtung mit dem Hydrogel (7) in Längsrichtung betrachtet maximal das distale Drittel des Ballons (3) umfasst.

3. Ballonkatheter nach Anspruch 2, **dadurch gekennzeichnet dass** die Beschichtung mit dem Hydrogel (7) auf die distale Spitze (8) des Ballonkatheters (1) beschränkt ist.

4. Ballonkatheter nach einem der Ansprüche 1 bis 3, **dadurch gekennzeichnet, dass** die Länge des mit dem Hydrogel (7) beschichteten Bereichs des Ballonkatheters (1) maximal 5 cm, bevorzugt maximal 3 cm, weiter bevorzugt maximal 2 cm beträgt.

5. Ballonkatheter nach einem der Ansprüche 1 bis 4, **dadurch gekennzeichnet, dass** die Menge an Hydrogel (7) pro beschichteter Flächeneinheit des Ballonkatheters (1) ≤ 2,5 µg/mm², weiter bevorzugt 0,5 bis 2,5 µg/mm² beträgt.

6. Ballonkatheter nach einem der Ansprüche 1 bis 5, **dadurch gekennzeichnet, dass** die Gesamtmenge an auf den Ballonkatheter (1) aufgebrachtem Hydrogel (7) ≤ 5 mg, vorzugsweise ≤ 3 mg, weiter vorzugsweise ≤ 1 mg beträgt.

7. Ballonkatheter nach einem der Ansprüche 1 bis 6, **dadurch gekennzeichnet, dass** das Hydrogel (7) frei von Wirkstoff ist.

8. Ballonkatheter nach einem der Ansprüche 1 bis 7, **dadurch gekennzeichnet, dass** das Hydrogel (7) ein Polysaccharid, ein modifiziertes Polysaccharid, ein Methacrylat, ein Polypeptid, ein Protein und/oder ein synthetisches Polymer enthält.

9. Ballonkatheter nach einem der Ansprüche 1 bis 8, **dadurch gekennzeichnet, dass** das Hydrogel (7) Dextran, Polyhydroxyethylmethacrylat, ein Polyacrylat, Chitosan, oxidierte Cellulose, Polyvinylpyrrolidon, Polyethylenglycol, Hyaluronsäure, Agarose, Stärke, Stärkederivate wie Hydroxyethylstärke, ein Poloxamer, Gelatine, Fibrin, Polyvinylalkohol, Propylenglycol, Polyacrylsäure, Methylcellulose, Carboxymethylcellulose, Hydroxypropylmethylcellulose, Hydroxyethylcellulose, Hydroxypropylcellulose, Alginat, Amylose, Amylopektin, Xanthan, Xyloglucan, Peptidoglycan, Polyoxyethylenderivate, ein Silikon und/oder Elastin enthält.

10. Ballonkatheter nach einem der Ansprüche 1 bis 9, **dadurch gekennzeichnet, dass** sich durch den Schaft (2) in Längsrichtung zumindest teilweise ein zweites Lumen (10) erstreckt, das der Aufnahme eines Führungsdrahts dient.

11. Ballonkatheter nach einem der Ansprüche 1 bis 10, **dadurch gekennzeichnet, dass** der Ballon (3) mit einem oder mehreren Wirkstoffen beschichtet ist.

12. Ballonkatheter nach Anspruch 11, **dadurch gekennzeichnet, dass** der verwendete Wirkstoff ausgewählt ist aus der Gruppe: Tretinoin, Orphanrezeptoragonisten, Elafinderivate, Corticosteroide, Steroidhormone, Paclitaxel, Rapamycin, Tacrolimus, hydrophobe Proteine, Heparin und/oder hormonartige oder zellproliferationsverändernde Substanzen.

13. Ballonkatheter nach einem der Ansprüche 1 bis 12, **dadurch gekennzeichnet, dass** sich an das proximale Ende des Schafts (6) ein oder mehrere Luer-Lock-Anschlüsse (4, 5) anschließen.

## Claims

1. Balloon catheter to be introduced into the blood vessel system of the human or animal body, said catheter having a longitudinally extending shaft (2), with the balloon catheter (1) comprising a distal portion (A) in which a balloon (3) is arranged that is expandable by the supply of a fluid through a lumen (9) extending through the shaft (2), wherein the distal tip (8) of the balloon catheter (1) is provided with a coating consisting of a hydrogel (7)
**characterized in that**
viewed in the longitudinal direction, the area of the coating with hydrogel (7) extends from the distal end of the balloon catheter (1) to maximally the middle of the balloon (3).

2. Balloon catheter according to claim 1, **characterized in that** the area of the coating with hydrogel (7), viewed in the longitudinal direction, maximally covers the distal third of the balloon (3).

3. Balloon catheter according to claim 2, **characterized in that** the coating with hydrogel (7) is confined to the distal tip (8) of the balloon catheter (1).

4. Balloon catheter according to any of claims 1 to 3, **characterized in that** the length of the area of the balloon catheter (1) coated with hydrogel (7) amounts to maximally 5 cm, preferably maximally 3 cm, further preferred maximally 2 cm.

5. Balloon catheter according to any of claims 1 to 4, **characterized in that** the amount of hydrogel (7) per coated unit area of the balloon catheter (1) amounts to ≤ 2.5 µg/mm², further preferably to 0.5 to 2.5 µg/mm².

6. Balloon catheter according to any of claims 1 to 5, **characterized in that** the total amount of hydrogel (7) applied to the balloon catheter (1) amounts to ≤ 5 mg, preferably ≤ 3 mg, further preferably ≤ 1 mg.

7. Balloon catheter according to any of claims 1 to 6, **characterized in that** the hydrogel (7) is free of active substances.

8. Balloon catheter according to any of claims 1 to 7, **characterized in that** the hydrogel (7) comprises a polysaccharide, a modified polysaccharide, a methacrylate, a polypeptide, a protein and/or a synthetic polymer.

9. Balloon catheter according to any of claims 1 to 8, **characterized in that** the hydrogel (7) comprises dextran, polyhydroxyethyl methacrylate, a polyacrylate, chitosan, oxidized cellulose, polyvinylpyrrolidone, polyethylene glycol, hyaluronic acid, agarose, starch, starch derivatives such as hydroxyethyl starch, a poloxamer, gelatin, fibrin, polyvinyl alcohol, propylene glycol, polyacrylic acid, methyl cellulose, carboxymethyl cellulose, hydroxypropyl methyl cellulose, hydroxyethyl cellulose, hydroxypropyl cellulose, alginate, amylose, amylopectin, xanthan, xyloglucan, peptidoglycan, polyoxyethylene derivatives, a silicone and/or elastin.

10. Balloon catheter according to any of claims 1 to 9, **characterized in that** a second lumen (10) extends at least partially through the shaft (2) in the longitudinal direction, said second lumen serving to accommodate a guidewire.

11. Balloon catheter according to any of claims 1 to 10, **characterized in that** the balloon (3) is coated with one or several active substances.

12. Balloon catheter according to claim 11, **characterized in that** the active substance or agent used is selected from the following group: Tretinoin, orphan receptor agonists, elafin derivatives, corticosteroids, steroid hormones, paclitaxel, rapamycin, tacrolimus, hydrophobic proteins, heparin and/or hormone-like or cell proliferation-modifying substances.

13. Balloon catheter according to any of claims 1 to 12, **characterized in that** one or several Luer lock connectors (4, 5) are arranged adjacent to the proximal end of the shaft (6).

## Revendications

1. Cathéter à ballonnet pour l'introduction dans le système vasculaire du corps humain ou animal avec une tige (2) s'étendant dans la direction longitudinale, le cathéter à ballonnet (1) disposant d'une section distale (A) dans laquelle est disposé un ballonnet (3) qui peut être dilaté par l'amenée d'un fluide via un lumen (9) s'étendant à travers la tige (2), la pointe distale (8) du cathéter à ballonnet (1) présentant un revêtement avec un hydrogel (7),
**caractérisé en ce que**
vue dans la direction longitudinale, la zone du revêtement avec l'hydrogel (7) s'étend de l'extrémité distale du cathéter à ballonnet (1) jusqu'au maximum au milieu du ballonnet (3).

2. Cathéter à ballonnet selon la revendication 1, **caractérisé en ce que** la zone d'hydrogel du revêtement avec l'hydrogel (7) comprend, vu dans le sens longitudinal, au maximum le tiers distal du ballonnet (3).

3. Cathéter à ballonnet selon la revendication 2, **caractérisé en ce que** le revêtement avec l'hydrogel (7) est limité à la pointe distale (8) du cathéter à ballonnet (1).

4. Cathéter à ballonnet selon l'une des revendications 1 à 3, **caractérisé en ce que** la longueur de la zone du cathéter à ballonnet (1) revêtue de l'hydrogel (7) est de 5 cm maximum, de préférence de 3 cm maximum, de préférence encore de 2 cm maximum.

5. Cathéter à ballonnet selon l'une des revendications 1 à 4, **caractérisé en ce que** la quantité d'hydrogel (7) par unité de surface revêtue du cathéter à ballonnet (1) est ≤ de 2,5 µg/mm², de préférence encore de 0,5 à 2,5 µg/mm².

6. Cathéter à ballonnet selon l'une des revendications 1 à 5, **caractérisé en ce que** la quantité totale d'hydrogel (7) appliquée sur le cathéter à ballonnet (1) est ≤ de 5 mg, de préférence ≤ de 3 mg, de préférence encore ≤ de 1 mg.

7. Cathéter à ballonnet selon l'une des revendications 1 à 6, **caractérisé en ce que** l'hydrogel (7) est exempt de substance active.

8. Cathéter à ballonnet selon l'une quelconque des revendications 1 à 7, **caractérisé en ce que** l'hydrogel (7) contient un polysaccharide, un polysaccharide modifié, un méthacrylate, un polypeptide, une protéine et/ou un polymère synthétique.

9. Cathéter à ballonnet selon l'une des revendications 1 à 8, **caractérisé en ce que** l'hydrogel (7) comprend du dextrane, du polyhydroxyéthylméthacrylate, un polyacrylate, du chitosane, de la cellulose oxydée, de la polyvinylpyrrolidone, du polyéthylèneglycol, de l'acide hyaluronique, de l'agarose, d'amidon, des dérivés d'amidon tels que l'hydroxyéthylamidon, un poloxamère, gélatine, fibrine, alcool polyvinylique, propylèneglycol, acide polyacrylique, méthylcellulose, carboxyméthylcellulose, hydroxypropylméthylcellulose, hydroxyéthylcellulose, hydroxypropylcellulose, alginate, amylase, amylopectine, xanthane, xyloglucane, peptidoglycane, dérivés de polyoxyéthylène, silicone et/ou élastine.

10. Cathéter à ballonnet selon l'une des revendications 1 à 9, **caractérisé en ce qu'**un deuxième lumen (10) s'étend longitudinalement à travers la tige (2), au moins partiellement, et sert à recevoir un fil de guidage.

11. Cathéter à ballonnet selon l'une des revendications 1 à 10, **caractérisé en ce que** le ballonnet (3) est revêtu d'une ou plusieurs substances actives.

12. Cathéter à ballonnet selon la revendication 11, **caractérisé en ce que** la substance active utilisée est choisie dans le groupe: trétinoine, agonistes des récepteurs orphelins, dérivés de l'élafine, corticostéroïdes, hormones stéro diennes, paclitaxel, rapamycine, tacrolimus, protéines hydrophobes, héparine et/ou substances de type hormonal ou modifiant la prolifération cellulaire.

13. Cathéter à ballonnet selon l'une des revendications 1 à 12, **caractérisé en ce qu'**un ou plusieurs raccords Luer-Lock (4, 5) se raccordent à l'extrémité proximale de la tige (6).
